# EUROPEAN PATENT APPLICATION

(11) **EP 3 769 800 A1**
(43) Date of publication of application: **27.01.2021**
(21) Application number: 18905906.6
(22) Date of filing: 30.11.2018
(51) Int. Cl.: A61M 5/14

(54) **LIQUID TRANSPORT CONTROL DEVICE AND LIQUID TRANSPORT APPARATUS**

(30) Priority: 21.03.2018 CN 201810235331
(71) Applicant: BOE Technology Group Co., Ltd., Beijing Anhui 100015 (CN); Hefei BOE Optoelectronics Technology Co., Ltd., Hefei, Anhui 230012 (CN)
(72) Inventor: LI, Peng, Beijing 100176 (CN); HUANG, Jiong, Beijing 100176 (CN); ZHANG, Zhen, Beijing 100176 (CN); HU, Lingxiao, Beijing 100176 (CN)
(74) Representative: Isarpatent
(86) International application number: PCT/CN2018/118518
(87) International publication number: WO 2019/179165

(57) **Abstract**

A liquid transfer control device and a liquid transfer apparatus are provided. The liquid transfer control device includes a tube carrier, configured to accommodate at least one portion of a tube, wherein the tube is made of a soft material; a flow rate sensor, configured to detect a flow rate of liquid transferred in the tube; a regulator, configured to regulate the flow rate of the liquid transferred in the tube; and a controller, configured to output a control signal to the regulator based on the flow rate detected by the flow rate sensor, wherein, the regulator is further configured to, in response to the control signal outputted by the controller, regulate the flow rate of the liquid in the tube accommodated in the tube carrier.

## Description

### CROSS-REFERENCE TO RELATED APPLICATION

This application claims a priority to Chinese Patent Application No. 201810235331.1 filed in China on March 21, 2018, the disclosure of which is incorporated in its entirety by reference herein.

### TECHNICAL FIELD

The present disclosure relates to the technical field of medical devices, and in particular relates to a liquid transfer control device and a liquid transfer apparatus.

### BACKGROUND

A transfer of blood or liquid medicine between an extracorporal environment and an intracorporal environment is extremely common in medical practice. For example, blood may be drawn from a body of a person when the person donates blood or when etiological diagnosis is performed by means of drawing blood; and blood needs to be transfused into a body of a person when the person is transfused with the blood, and liquid medicine needs to be transfused into a body of a person when the person is subject to a medical treatment.

In related art, it is common to directly connect the body with a tube for liquid transfer when liquid transfers as mentioned above are performed, a condition of a liquid transfer is incapable of being monitored and a medical accident may occur due to the incapability of monitoring the condition of the liquid transfer.

Therefore, when a liquid transfer between the extracorporal environment and the intracorporal environment is performed, liquid flowing in the extracorporal environment needs to be monitored and controlled so as to avoid abnormality of a flow rate of the liquid and further prevent pain from being brought to a patient or even prevent a medical accident from happening.

### SUMMARY

Some embodiments of the present disclosure provide a liquid transfer control device and a liquid transfer apparatus.

In a first aspect, a liquid transfer control device is provided in the present disclosure. The liquid transfer control device includes a tube carrier, configured to accommodate at least one portion of a tube, wherein the tube is made of a soft material; a flow rate sensor, configured to detect a flow rate of liquid transferred in the tube; a regulator, configured to regulate the flow rate of the liquid transferred in the tube; and a controller, coupled to the regulator and configured to output a control signal to the regulator based on the flow rate detected by the flow rate sensor, wherein, the regulator is further configured to, in response to the control signal outputted by the controller, regulate the flow rate of the liquid in the tube accommodated in the tube carrier.

Optionally, the tube carrier further includes a tube regulating mechanism, the tube regulating mechanism include a fluid container and an accommodation space, the accommodation space is arranged in the middle of the fluid container and configured to accommodate the at least one portion of the tube; and the fluid container includes a first surface facing towards the tube and made of an elastically deformable material.

Optionally, the regulator includes a gas-pressure regulator, the gas-pressure regulator is arranged in the tube carrier and configured to regulate a cross-sectional area of the tube arranged in the accommodation space by regulating a gas pressure within the fluid container.

Optionally, the gas-pressure regulator includes an air pump, a pressure regulating valve, and a gas transfer tube, the gas transfer tube has a first end communicated to an interior of the fluid container and a second end communicated to the air pump, wherein the pressure regulating valve is arranged on the gas transfer tube, and the air pump and the pressure regulating valve are configured to regulate the cross-sectional area of the tube by regulating the gas pressure within the fluid container.

Optionally, the fluid container is filled with liquid, the regulator includes a hydraulic regulator, the hydraulic regulator is arranged in the tube carrier and configured to regulate the cross-sectional area of the tube arranged in the accommodation space by regulating a pressure of the liquid within the fluid container.

Optionally, the hydraulic regulator includes a liquid storage tank, a hydraulic pump, a hydraulic value, a first liquid transfer tube, and a second liquid transfer tube; the hydraulic pump is connected to the liquid storage tank; the first liquid transfer tube has a first end communicated to the hydraulic pump and a second end communicated to an interior of the fluid container; the second liquid transfer tube has a first end communicated to the liquid storage tank and a second end communicated to the interior of the fluid container; the hydraulic valve is arranged on the second liquid transfer tube; and the hydraulic pump and the hydraulic valve are configured to regulate the cross-sectional area of the tube by regulating the pressure of the liquid within the fluid container.

Optionally, the controller is further configured to output the control signal to the regulator in response to a detection result that the flow rate detected by the flow rate sensor falls outside a first predefined value range.

Optionally, the liquid transfer control device further includes a first alarm, the first alarm is arranged on the tube carrier and configured to send first alarm prompt information; wherein the controller is further configured to, in response to a detection result that the flow rate detected by the flow rate sensor falls outside the first predefined value range, send a first alarm signal to the first alarm, the first alarm is further configured to send the first alarm prompt information in response to the first alarm signal.

Optionally, the liquid transfer control device further includes a physical sign detector configured to detect physical sign information of a human body connected to the tube; wherein the controller is further configured to acquire the physical sign information detected by the physical sign detector, and output the control signal to the regulator based on the physical sign information, so that the regulator regulates the cross-sectional area of the tube accommodated in the tube carrier in response to the control signal.

Optionally, the controller is further configured to output the control signal to the regulator in response to a detection result that the physical sign information detected by the physical sign detector falls outside a second predefined value range.

Optionally, the liquid transfer control device further includes a second alarm, the second alarm is configured to send second alarm prompt information; wherein the controller is further configured to, in response to the detection result that the physical sign information detected by the physical sign detector falls outside the second predefined value range, send a second alarm signal to the second alarm; the second alarm is further configured to send the second alarm prompt information in response to the second alarm signal.

Optionally, the physical sign detector is arranged on a wearing member, the wearing member is fixable onto a human body, and in a case that the wearing member is fixed to the human body, the physical sign detector is capable of detecting the physical sign information of the human body. The liquid transfer control device further includes a first wireless transceiver coupled to the physical sign detector and arranged on the wearing member; and a second wireless transceiver coupled to the controller and arranged on the tube carrier; wherein the physical sign information detected by the physical sign detector is transmitted to the second wireless transceiver via the first wireless transceiver and the controller acquires the physical sign information from the second wireless transceiver.

Optionally, the liquid transfer control device further includes a display configured to acquire and display the flow rate detected by the flow rate sensor and the physical sign information detected by the physical sign detector.

Optionally, the display is a touch display screen and configured to enable a user to input at least one of the first predefined value range of the flow rate or the second predefined value range of the physical sign information.

Optionally, the tube is a blood transfer tube used for drawing blood or blood donation, or the tube is a liquid-medicine transfer tube used to transfuse liquid medicine to a human body.

Optionally, at least a portion of the flow rate sensor is inserted into the fluid container; or the flow rate sensor is arranged outside the fluid container.

Optionally, the physical sign sensor includes at least one of a body temperature detector, a pulse detector, or a blood pressure detector.

Optionally, the wearing member further includes an auxiliary controller, the auxiliary controller is configured to be coupled to the first wireless transceiver and to communicate with the controller via the first wireless transceiver.

Optionally, the regulator includes a cross-section regulator, the cross-section regulator is configured to regulate the flow rate of the liquid in the tube in response to the control signal from the controller.

Optionally, the cross-section regulator includes a pneumatic actuator, an air pump, and a gas transfer tube, the gas transfer tube connects the pneumatic actuator with the air pump, and the air pump is configured to control the pneumatic actuator to regulate the cross-sectional area of the tube in response to the control signal from the controller; or the cross-section regulator includes a hydraulic regulator, a hydraulic pump, and a liquid transfer tube, the liquid transfer tube connects the hydraulic regulator with the hydraulic pump, and the hydraulic pump is configured to control the hydraulic regulator to regulate the cross-sectional area of the tube in response to the control signal from the controller; or the cross-section regulator includes an electric actuator and a motor, the motor is configured to control the electric actuator to regulate the cross-sectional area of the tube in response to the control signal from the controller.

In a second aspect, a liquid transfer apparatus is provided in the present disclosure, the liquid transfer apparatus includes the liquid transfer control device according to the first aspect; and a tube made of a soft material and configured for transferring liquid, wherein at least one portion of the tube is accommodated in the tube carrier.

### BRIEF DESCRIPTION OF THE DRAWINGS

FIG. 1A is a schematic diagram showing a liquid transfer control device according to some embodiments of the present disclosure;
FIG. 1B is a sectional view taken along a line A-A' in FIG 1A;
FIG. 2 is a schematic diagram showing a liquid transfer control device according to some embodiments of the present disclosure;
FIGs. 3A to 3E are schematic views showing structures of a regulator in the liquid transfer control device according to some embodiments of the present disclosure;
FIG. 4 is a first schematic diagram showing a partial structure of the liquid transfer control device according to some embodiments of the present disclosure;
FIG. 5 is a second schematic diagram showing a partial structure of the liquid transfer control device according to some embodiments of the present disclosure; and
FIG. 6 is a schematic diagram showing a structure of a liquid transfer apparatus according to some embodiments of the present disclosure.

### DETAILED DESCRIPTION

To make technical problems, technical solutions, and advantages of the present disclosure more apparent, detailed description will be made in conjunction with accompanying drawings and embodiments.

A liquid transfer control device and a liquid transfer apparatus provided by the present disclosure are capable of monitoring a condition of liquid transfer between an extracorporal environment and an intracorporal environment, so that a transfer rate of the liquid transfer may be controlled as appropriated, and a medical accident may be prevented from occurring due to abnormality of the transfer rate.

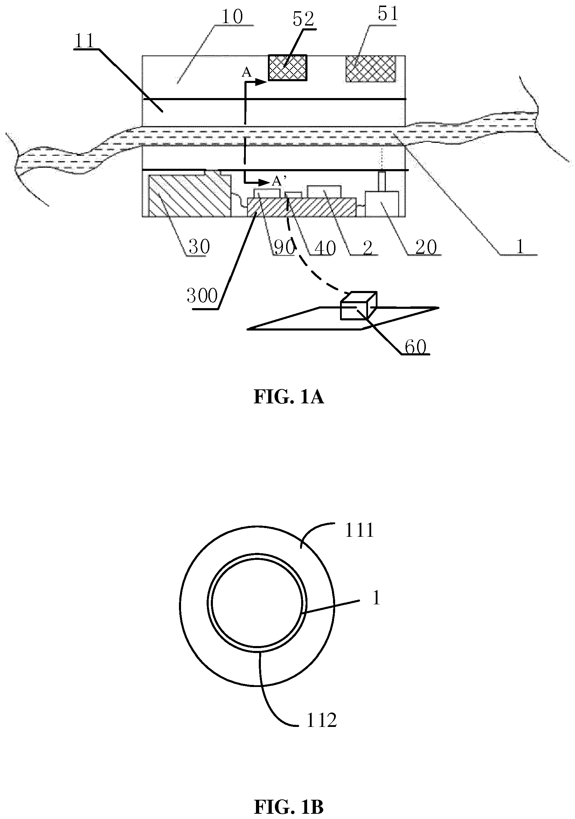

FIG. 1A is a schematic diagram showing a liquid transfer control device according to some embodiments of the present disclosure. Referring to FIG. 1A, the liquid transfer control device includes a tube carrier 10, a flow rate sensor 20, a regulator 30, and a controller 40. The tube carrier 10 is configured to have an interior through which a tube 1 made of a soft material and used for liquid transfer is disposed to pass, and the tube carrier 10 includes a tube regulating mechanism 11. The tube regulating mechanism 11 includes a fluid container 111 and an accommodation space 112. The accommodation space 112 is located in the middle of the fluid container 111 and configured to accommodate at least one portion of the tube 1. The flow rate sensor 20 is configured to detect a flow rate of liquid transferred in the tube 1. The regulator 30 is configured to regulate the flow rate of the liquid transferred in the tube 1 by regulating a cross-sectional area of the tube 1 arranged in the tube regulating mechanism 11. The controller 40 is configured to output a control signal to the regulator 30 based on the flow rate detected by the flow rate sensor 20 such that the regulator 30 regulates the cross-sectional area of the tube 1 arranged in the tube regulating mechanism 11.

Referring to FIG. 1B, FIG. 1B is a cross-sectional view taken along a line A-A' in FIG. 1A. A cross section of the tube regulating mechanism 11 taken along a direction perpendicular to an extending direction of the tube 1 may have a circular ring shape. The tube regulating mechanism 11 includes a first surface facing towards the tube 1. The first surface may be made of an elastically deformable material. The elastically deformable material protrudes towards the tube 1 or caves backwards from the tube 1 with a change in a fluid pressure of fluid contained in the fluid container 111, so as to control a size of the cross-sectional area of the tube 1. The tube 1 is accommodated in the accommodation space 112 defined by the first surface of the tube regulating mechanism 111. Optionally, a cross-sectional area of the accommodation space 112 is larger than that of the tube 1.

In the liquid transfer control device according to the embodiments of the present disclosure, the tube is provided to pass through the tube carrier, and the regulator is capable of regulating the cross-sectional area of the tube in the tube regulating mechanism of the tube carrier to achieve a purpose of regulating the flow rate in the tube; moreover, based on the flow rate of the liquid transferred in the tube obtained by monitoring the flow rate by the flow rate sensor, the controller may control the regulator to regulate the cross-sectional area of the tube, so as to regulate the flow rate of the liquid transferred in the tube.

In some embodiments of the present disclosure, the flow rate of the liquid in the tube, as mentioned herein, refers to a flow velocity of the liquid in the tube. It may be understood that when the cross-sectional area of the tube arranged in the tube regulating mechanism is increased by the regulator, the flow rate of the liquid in the entirety of the tube is increased, thus increasing an amount of the liquid transfused into or drawn out of a human body per unit time; and when the cross-sectional area of the tube arranged in the tube regulating mechanism is reduced by the regulator, the flow rate of the liquid in the entirety of the tube is decreased, thus reducing the amount of the liquid transfused into or drawn out of the human body per unit time.

Therefore, the liquid transfer control device according to some embodiments of the present disclosure is capable of monitoring the condition of liquid transfer between the extracorporal environment and the intracorporal environment, and controlling the flow rate of the liquid transfer as appropriate to avoid a medical accident due to the abnormality of the flow rate.

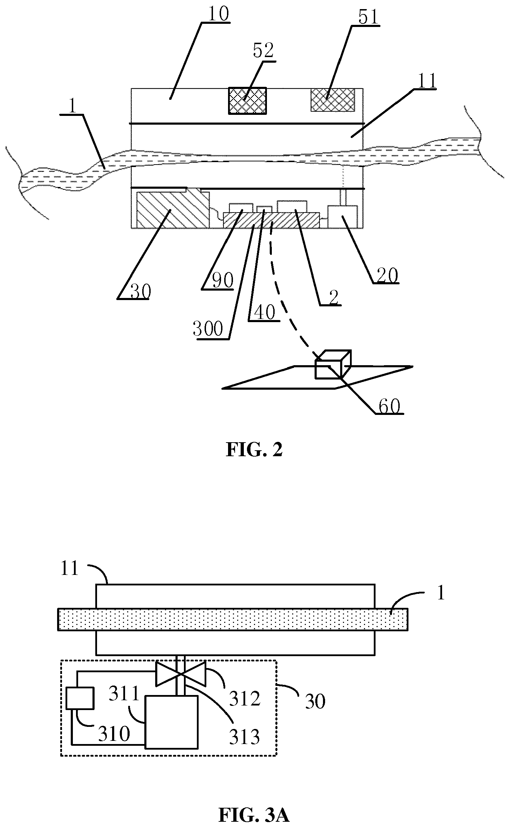

Referring to FIG. 2, FIG. 2 shows another schematic diagram of a liquid transfer control device according to some embodiments of the present disclosure. According to a principle that the flow rate of the liquid in the tube 1 is directly proportional to the cross-sectional area of the tube 1, when the cross-sectional area of the tube 1 arranged in the tube regulating mechanism 11 of the tube carrier 10 is decreased, the flow rate of the liquid in the tube 1 will be reduced; on the contrary, when the cross-sectional area of the tube 1 arranged in the tube regulating mechanism 11 of the tube carrier 10 is increased, the flow rate of the liquid in the tube 1 will be increased. Therefore, when the flow rate sensor 20 monitors that the flow rate of the liquid transferred in the tube 1 is abnormal, the cross-sectional area of the tube 1 arranged in the tube regulating mechanism 11 may be adjusted to change the flow rate of the liquid transferred in the tube 1, so as to achieve the purpose of controlling the flow rate of the liquid transferred in the tube 1 in real time.

The liquid transfer control device may be applied to a blood transfer tube used for drawing blood or donating blood or may be used to a liquid-medicine transfer tube used for transfusing liquid medicine to a human body. Through monitoring a condition of the liquid transfer between the extracorporal environment and the intracorporal environment, and the flow rate of the liquid may be controlled as appropriate so as to avoid a medical accident caused due to abnormality of the flow rate.

In the liquid transfer control device according to some embodiments of the present disclosure, the regulator 30 includes a gas-pressure regulator. The gas-pressure regulator may receive a control signal from the controller 40, and regulate a gas pressure in the tube regulating mechanism 11 based on the control signal so as to regulate the cross-sectional area of the tube 1 arranged in the tube regulating mechanism 11.

Optionally, referring to FIG. 3A, the gas-pressure regulator may include an air pump 311, a pressure regulating valve 312, and a gas transfer tube 313. The gas transfer tube 313 has a first end communicated to an interior of a fluid container of the tube regulating mechanism 11, and a second end communicated to the air pump 311. The pressure regulating valve 312 is provided on the gas transfer tube 313. The air pump 311 and the pressure regulating valve 312 are also coupled to the controller 40, and change the cross-sectional area of the tube 1 in the tube regulating mechanism 11 by regulating a gas pressure within the fluid container 111. As shown in FIG. 2, when the gas pressure in the fluid container 111 is increased, the tube 1 is squeezed to have a smaller cross-sectional area; and when the gas pressure in the fluid container 111 is decreased, the cross-sectional area of the tube 1 is increased.

In the liquid transfer control device according to some embodiments of the present disclosure, the tube regulating mechanism 11 is filled with liquid, and the regulator 30 includes a hydraulic regulator. The hydraulic regulator may receive a control signal from the controller 40 and regulate the cross-sectional area of the tube 1 arranged in the tube regulating mechanism 11 by regulating a pressure of liquid in the fluid container 111 in response to the control signal.

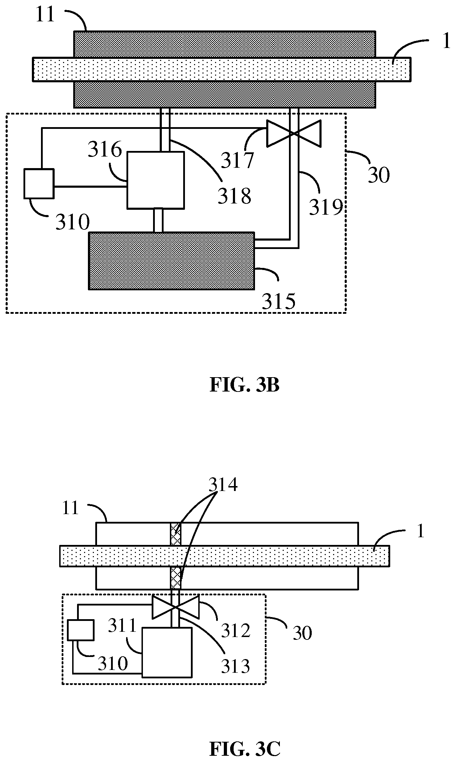

Optionally, referring to FIG. 3B, the hydraulic regulator may include a liquid storage tank 315, a hydraulic pump 316, a hydraulic valve 317, a first liquid transfer tube 318, and a second liquid transfer tube 319. The hydraulic pump 316 is connected to the liquid storage tank 315 and communicated to an interior of the fluid container 111 through the first liquid transfer tube 318. The liquid storage tank 315 is also communicated to the interior of the fluid container 111 through the second liquid transfer tube 319, and the hydraulic valve 317 is provided on the second liquid transfer tube 319. The hydraulic pump 316 and the hydraulic valve 317 are coupled to the controller 40. The hydraulic pump is configured to draw liquid from the liquid storage tank and pumps the liquid into the interior of the fluid container 111 through the first liquid transfer tube 318 to increase the liquid pressure within the fluid container 111 and reduce the cross-sectional area of the tube 1 in the tube regulating mechanism 11. Furthermore, a liquid pressure controlled by the hydraulic valve 317 may also be regulated, so that the liquid in the fluid container 111 may flow back to the liquid storage tank 315 through the second liquid transfer tube 319, so that the liquid pressure within the fluid container 111 is reduced, and the cross-sectional area of the tube 1 in the tube regulating mechanism 11 is increased. Therefore, with the hydraulic regulator having the above configuration, the liquid pressure within the fluid container 111 may be regulated to change the cross-sectional area of the tube 1 in the tube regulating mechanism 11.

Optionally, referring to FIGS. 3A and 3B, a second controller 310 coupled to the controller 40 may also be provided in the regulator 30. In a case that the regulator 30 includes the air pump 311 and the pressure regulating valve 312, the second controller may be coupled to the pressure regulating valve 312 and the air pump 11 so as to control, according to a control signal received from the controller 40, the air pump 311 and the pressure regulating valve 312 to regulate the gas pressure within the tube regulating mechanism 11, therefore changing the cross-sectional area of the tube 1 in the tube regulating mechanism 11. In a case that the regulator 30 includes the hydraulic pump 316 and the hydraulic valve 317, the second controller may be coupled to the hydraulic pump 316 and the hydraulic valve 317 so as to control, according to a control signal received from the controller 40, the hydraulic pump 316 and the hydraulic valve 317 to regulate liquid pressure of the liquid within the tube regulating mechanism 11, therefore changing the cross-sectional area of the tube 1 in the tube regulating mechanism 11.

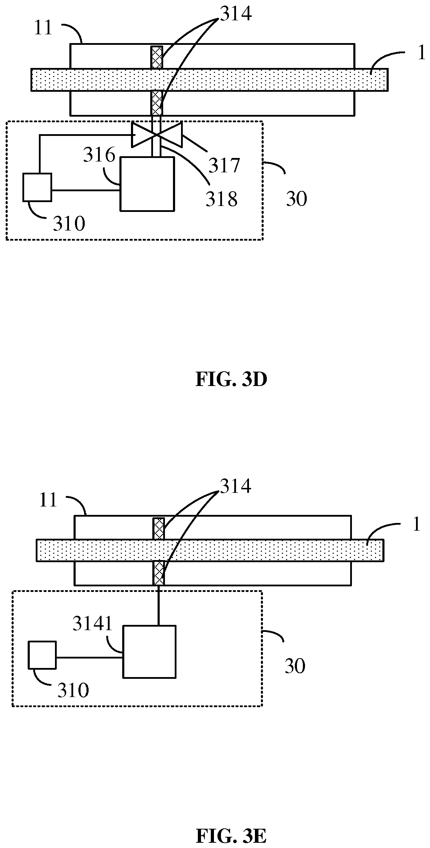

Optionally, in some embodiments of the present disclosure, as shown in FIGs. 3C and 3D, the regulator 30 may further include a cross-section regulator 314. In a case that the regulator 30 includes the gas-pressure regulator, the cross-section regulator 314 may be a pneumatic actuator and configured to change the cross-sectional area of the tube 1 as a function of the gas pressure in the gas transfer tube 313. In a case that the regulator 30 includes the hydraulic regulator, the cross-section regulator 14 may be a hydraulic actuator and configured to change the cross-sectional area of the tube 1 as a function of the liquid pressure of the liquid within the hydraulic transfer tube 318. In this case, the regulator 30 may only include the cross-section regulator 314, the hydraulic pump 316, the hydraulic valve 317, and the first liquid transfer tube 318.

Optionally, referring to FIG. 3E, the cross-section regulator 314 may be an electrically-driven component and configured to change the cross-sectional area of the tube 1 under an action of a motor 3141. In this case, the motor 3141 may be directly coupled to the controller 40 or to the second controller 310 so as to change the cross-sectional area of the tube 1 under a control of the controller 40 or the second controller 310.

In the liquid transfer control device according to some embodiments of the present disclosure, the flow rate sensor 20 includes an ultrasonic flow rate sensor. In particular, the ultrasonic flow rate sensor detects the flow rate of the liquid transferred in the tube 1 by transmitting ultrasonic waves to the interior of the tube 1. Those skilled in the art should be able to understand a principle of detecting the flow rate by using the ultrasonic flow rate sensor, which will not be described in detail herein.

In some embodiments of the present disclosure, optionally, as shown in FIG. 1A, the ultrasonic flow rate sensor is provided on the tube carrier 10 and configured to detect the flow rate of liquid transferred in the tube 1 arranged in the tube regulating mechanism 11. Naturally, in a stable state, the flow rate at every position in the tube 1 is identical, and the ultrasonic flow rate sensor may not only detect the flow rate of liquid in the tube 1 arranged in the tube regulating mechanism 11 only, but also detect the flow rate of liquid in the tube 1 arranged outside the tube carrier 10 and obtain the flow rate of the liquid in the tube 1. Therefore, the ultrasonic flow rate sensor may be positioned independently of the tube carrier 10.

In conjunction with FIGs. 1A and 2, in a case that the ultrasonic flow rate sensor is provided on the tube carrier 10, since the ultrasonic flow rate sensor needs to detect the flow rate of the liquid in the tube 1 arranged in the tube regulating mechanism 11, at least a portion of the ultrasonic flow rate sensor may be optionally inserted into the interior of the tube regulating mechanism 11 so that ultrasonic waves may be emitted into the tube 1 arranged in the tube regulating mechanism 11. Optionally, a wall surface forming the tube regulating mechanism 11 of the tube carrier 10 is made of a light-transmissible material so that the ultrasonic wave emitted from the ultrasonic flow rate sensor may be emitted into the tube 1 through the wall surface of the tube carrier 10.

In some embodiments of the present disclosure, optionally, the controller 40 may be a Microcontroller Unit (MCU) or a microcontroller. The controller 40 is coupled to each of the regulator 30 and the flow rate sensor 20 and is configured to acquire a flow rate signal detected by the flow rate sensor 20 and output a control signal to the regulator 30.

Optionally, each of the controller 40 and the regulator 30 is arranged on the tube carrier 10. The flow rate sensor 20 may also be arranged on the tube carrier 10.

In some embodiments of the present disclosure, the controller 40 is configured to output a control signal to the regulator 30 in response to a detection result that the flow rate detected by the flow rate sensor 20 falls outside of a first predefined value range.

Specifically, when drawing or donating blood through the tube 1, the first predefined value range may be a first conventional flow-rate range of blood flowing in the tube 1 predefined for a case of drawing blood or donating blood; and when transfusing liquid medicine into the human body through the tube 1, the first predefined value range may be a second conventional flow-rate range of the liquid medicine in the tube 1 predefined for a case of transfusing liquid medicine. The controller 40 compares the flow rate detected by the flow rate sensor with the first predefined value range. In a case that a result of the comparison indicates that the flow rate detected by the flow rate sensor falls outside of the first predefined value range, the controller 40 determines that the flow rate of the liquid in the tube 1 is abnormal and outputs a control signal to the regulator 30. The regulator 30 then regulates the flow rate of the liquid transferred in the tube 1 by regulating the cross-sectional area of the tube 1 arranged in the tube regulating mechanism 11, so as to control the flow rate of the liquid in the tube 1 within the first predefined value range, thereby avoiding a medical accident caused by the abnormality of the flow rate.

More specifically, in a case that the flow rate detected by the flow rate sensor 20 exceeds a maximum value of the first predefined value range, a control signal is output to the regulator 30 to cause the regulator 30 to reduce the cross-sectional area of the tube 1 in the tube regulating mechanism 11; and in a case that the flow rate detected by the flow rate sensor 20 is below a minimum value of the first predefined value range, a control signal is outputted to the regulator 30 to cause the regulator 30 to increase the cross-sectional area of the tube 1 in the tube regulating mechanism 11.

Optionally, in some embodiments of the present disclosure, as shown in FIGs. 1A and 2, the liquid transfer control device further includes a first alarm 51 configured to send first alarm prompt information. The controller 40 is further configured to, in response to a detection result that the flow rate detected by the flow rate sensor 20 falls outside of the first predefined value range, send an alarm signal to the first alarm 51 so as to cause the first alarm 51 to send the first alarm prompt information.

Optionally, the first alarm 51 may include at least one of a sound alarm and a light alarm. In a case that the controller 40 determines that the flow rate detected by the flow rate sensor 20 falls outside of the conventional setting ranges, the controller 40 controls the first alarm 51 to give an alarm so as to prompt a medical staff to respond in time.

Optionally, as shown in FIGs. 1A and 2, the liquid transfer control device according to some embodiments of the present disclosure further includes a physical sign detector 60 configured to detect physical sign information of a human body to which the tube 1 is connected. The controller 40 is further configured to acquire the physical sign information detected by the physical sign detector 60, and output a control signal to the regulator 30 based on the physical sign information so that the regulator 30 regulates the cross-sectional area of the tube 1 arranged in the tube regulating mechanism 11.

Optionally, the physical sign detector 60 includes at least one of a pulse detector or a blood pressure detector. The pulse detector is configured to detect a pulse rate of a human body to which the tube 1 is connected, and the blood pressure detector is configured to detect a blood pressure value of the human body to which the tube 1 is connected.

The physical sign detector 60 may not only include the above-mentioned detectors capable of detecting the physical sign information of the human body, but also include, for example, a body temperature detector for detecting a body temperature of the human body to which the tube 1 is connected.

By providing the physical sign detector 60 coupled to the controller 40, the liquid transfer control device may control, based on the physical sign information of the human body detected by the physical sign detector 60, the regulator 30 to regulate the cross-sectional area of the tube 1 arranged in the tube regulating mechanism 11, thus avoiding a danger from being incurred to a human life due to continuing to draw blood, donate blood or transfuse liquid medicine through the tube 1 at the conventional flow rates under a condition that the human body is uncomfortable (for example, an abnormal physical sign such as an excessively fast heart rate is generated).

Specifically, the controller 40 is configured to output a control signal to the regulator 30 in response to a detection result that the physical sign information detected by the physical sign detector 60 falls outside of a second predefined value range.

The second predefined value range is a normal value range of the physical sign information detected by the physical sign detector 60. The controller 40 outputs a control signal to the regulator 30 in a case that the detected physical sign information is outside of the normal value range. In some embodiments of the present disclosure, in a case that the detected physical sign information is outside of the normal value range, the control signal is output to the regulator 30 so that the regulator 30 is caused to reduce the cross-sectional area of the tube 1 arranged in the tube regulating mechanism 11, thereby avoiding discomfort caused to the human body by an excessively fast flow rate of the liquid in the tube 1, or the regulator 30 is caused to increase the cross-sectional area of the tube 1 arranged in the tube regulating mechanism 11, thereby avoiding discomfort caused to the human body by an excessively slow flow rate of the liquid in the tube 1.

Taking as an example a case in which the physical sign detector 60 includes the pulse detector and liquid medicine is transfused to the human body through the tube 1, in a case that the pulse detector detects that a current pulse rate of the human body exceeds a maximum value of the second predefined value range, the case indicates that an abnormal heart rate of the human body currently happens in a process of transfusing the liquid medicine, the controller 40 outputs a control signal to the regulator 30 to cause the regulator 30 to reduce the cross-sectional area of the tube 1 arranged in the tube regulating mechanism 11.

Taking as an example a case that the physical sign detector 60 includes the blood pressure detector configured to detect a blood pressure of the human body, in a case that the blood pressure detector detects an abnormal blood pressure, the controller 40 instructs the regulator 30 to regulate the cross-sectional area of the tube 1 in the tube regulating mechanism 11, for example, to increase or reduce the cross-sectional area of the tube 1 in the tube regulating mechanism 11, so as to avoid discomfort of the human body caused by an excessively fast or slow flow rate of the liquid in the tube 1.

In some embodiments of the present disclosure, optionally, the liquid transfer control device further includes a second alarm 52 configured to send second alarm prompt information. The controller 40 is further configured to, in response to a detection result that the physical sign information detected by the physical sign detector 60 falls outside of the second predefined value range, send an alarm signal to the second alarm 52 so as to cause the second alarm 52 to send the second alarm prompt information.

Optionally, the second alarm 52 may include at least one of a sound alarm or a light alarm. In a case that the controller 40 determines that the physical sign information detected by the physical sign detector 60 is abnormal, the controller 40 controls the second alarm 52 to give an alarm to prompt a medical staff to respond in time, so as to avoid a dangerous situation from occurring. Optionally, the first alarm 51 and the second alarm 52 may be configured to emit different sounds or different colors of light, so as to enable the medical staff to distinguish the abnormality of the physical sign information detected by the physical sign detector 60 from the abnormality of the flow rate detected by the flow rate sensor 20.

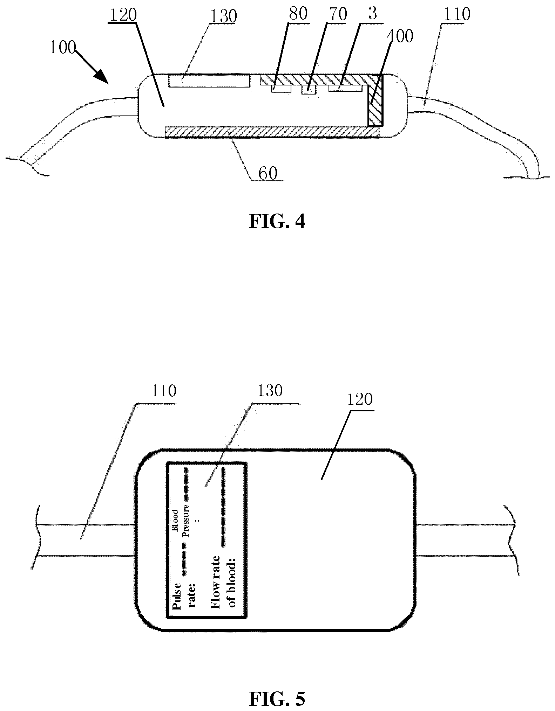

Based on the physical sign detector 60 as provided above, the liquid transfer control device according to some embodiments of the present disclosure further includes at least one wearing member 100, as shown in FIGs. 4 and 5. The physical sign detector 60 is arranged on the at least one wearing member 100. The at least one wearing member 100 may be fixed to the human body. Under a condition that the wearing member 100 is fixed to the human body, the physical sign detector 60 may detect physical sign information of the human body, thus detecting the physical signs of the human body.

Taking as an example the case that the physical sign detector 60 includes the pulse detector, the at least one wearing member 100 is formed to be a wristband member. The at least one wearing member may include a wristband 110 capable of being worn on a wrist of the human body and a carrying portion 120 provided on the wristband 110, wherein the physical sign detector 60 is arranged on the carrying portion 120. Under a condition that the wristband 110 is worn on the wrist of the human body, the pulse detector on the carrying portion 120 is in contact with the skin of the wrist of the human body and may detect a pulse rate.

Optionally, the second alarm 52 coupled to the controller 40 of FIG. 1A is arranged on the carrying portion 120. Of course, it may be understood that the second alarm 52 may also be arranged on the tube carrier 10 instead, or the second alarm 52 may be combined with the first alarm 51 and form a single same structure.

Optionally, in some embodiments of the present disclosure, as shown in FIG. 4, the liquid transfer control device further includes a first wireless transceiver 80 and a second wireless transceiver 90. The first wireless transceiver 80 is coupled to the physical sign detector 60 and may be arranged on the carrying portion 120. The second wireless transceiver 90 is coupled to the controller 40 and may be arranged on the tube carrier 10, as shown in FIG. 1A. For example, each of the first wireless transceiver 80 and the second wireless transceiver 90 may be a Bluetooth transceiver, an infrared transceiver, a Wireless Fidelity (Wi-Fi) transceiver, or a ZigBee transceiver.

The physical sign information detected by the physical sign detector 60 may be transmitted to the second wireless transceiver 90 through the first wireless transceiver 80, and the controller 40 then acquires the physical sign information.

Optionally, referring to FIGs. 1A and 2, the liquid transfer control device further includes a bus 300. The bus 300 is arranged on the tube carrier 10 and configured to couple the flow rate sensor 20, the regulator 30, the first alarm 51, and the second wireless transceiver 90 to the controller 40 so that signals are transmitted among the flow rate sensor 20, the regulator 30, the first alarm 51, the second wireless transceiver 90 and the controller 40. For example, the controller 40 receives a flow rate signal from the flow rate sensor 20, the controller 40 outputs a control signal to the regulator 30, and the controller 40 sends an alarm signal to the first alarm 51, receives a signal from the second wireless transceiver 90, transmits a signal to the second wireless transceiver 90, etc. The bus 300 may be any bus structure 300 known in the art, which may be used for communication among the above elements.

Based on the above arrangement of elements, in the liquid transfer control device according to some embodiments of the present disclosure, the tube carrier 10 is arranged separately from and in wireless communication with the wearing member 100. During use, the wearing member 100 is tied to a human body and is configured to detect physical sign information of the human body; and the tube passes through the tube carrier 10 and is used for drawing blood, blood donation or liquid medicine transfusion related to the human body. In a case that the physical sign detector 60 in the wearing device 100 detects physical sign information of the human body, the physical sign information is transmitted to the controller 40 arranged on the tube carrier 10 through the wireless communication between the first wireless transceiver 80 and the second wireless transceiver 90, so that the controller 40 sends a control signal to the regulator 30 based on the physical sign information of the human body to regulate the cross-sectional area of the tube 1 arranged in the tube regulating mechanism 11.

In addition, in a case that the second alarm 52 coupled to the controller 40 is arranged on the carrying portion 120, the controller 40 and the second alarm 52 may be wirelessly connected to each other via the second wireless transceiver 90 and the first wireless transceiver 80. That is, the controller 40 transmits an alarm signal to the second alarm 52 via the second wireless transceiver 90 and the first wireless transceiver 80, causing the second alarm 52 to generate an alarm.

Optionally, in some embodiments of the present disclosure, as shown in FIGs. 4 and 5, the liquid transfer control device further includes a display 130 for acquiring and displaying the flow rate detected by the flow rate sensor 20 and the physical sign information detected by the physical sign detector 60.

Optionally, the display 130 may be a touchable display screen such as a touch display screen. The touchable display screen is configured to enable a user to set at least one of the first predefined value range or the second predefined value range. In the present disclosure, the user may input the first predefined value range or the second predefined value range through the display 130, so that different parameters may be set for different users to meet customization requirements of the user. Of course, at least one of the first predefined value range or the second predefined value range may also be predefined by a manufacturer of the liquid transfer control device of the present disclosure when the manufacture produces the liquid transfer control device, or may be set by the user by means of an external device such as by means of a computer or a mobile phone APP through the first wireless transceiver 80 or the second wireless transceiver 90.

Optionally, in some embodiments of the present disclosure, the display 130 is integrated with the physical sign detector 60. As shown in FIGs. 4 and 5, each of the display 130 and the physical sign detector 60 is arranged on the wearing member 100. The controller 40 may be connected to the display 130 through the first wireless transceiver 80 and the second wireless transceiver 90, and send the flow rate detected by the flow rate sensor 20 to the display 130 for display.

Optionally, the display 130 may be arranged on the tube carrier 10.

In some embodiments of the present disclosure, a structure of the liquid transfer control device provided in some embodiments of the present disclosure is illustrated in detail by taking as an example a case in which each of the physical sign detector 60, the second alarm 52, and the display 130 arranged on the wearing member 100 is in wireless communication with the controller 40 arranged on the tube carrier 10 via the first wireless transceiver 80 and the second wireless transceiver 90. It may be understood that an auxiliary controller 70 may also be provided on the wearing member 100. The auxiliary controller 70 is communicatively connected with the controller 40 through the first wireless transceiver 80 and the second wireless transceiver 90, and is connected to each of the physical sign detector 60, the second alarm 52, and the display 130. The auxiliary controller 70 is configured to acquire physical sign information detected by the physical sign detector 60 and send the physical sign information to the controller 40; and is configured to acquire an alarm signal sent by the controller 40 and send the alarm signal to the second alarm 52; and is configured to acquire flow rate information sent by the controller 40 and send the flow rate information to the display 130 for display. Moreover, the auxiliary controller 70 may also send the physical sign information detected by the physical sign detector 60 to the display 130 for display. Optionally, the auxiliary controller 70 may further have a calculating function, and after the auxiliary controller 70 acquires the physical sign information detected by the sign detector 60, the auxiliary controller 70 may compare the physical sign information with the second predefined value range. In a case that the auxiliary controller 70 determines that the physical sign information is outside of the second present value range, the auxiliary controller 70 may transmit to the controller 40 a determination result that the physical sign information is outside of the second predefined value range so that the controller 40 controls the regulator 30 to regulate the cross-sectional area of the tube 1.

Optionally, the auxiliary controller 70 or the second controller 310 may be a Micro Controller Unit (MCU) or a microcontroller.

Optionally, referring to FIG. 4, the wearing member 100 further includes a bus 400. The bus 400 is configured to connect the first wireless transceiver 80, the physical sign detector 60, the auxiliary controller 70, and the display 130 with each other such that signals are transmitted between the first wireless transceiver 80, the physical sign detector 60, the auxiliary controller 70, and the display 130. The bus 400 may be any bus structure known in the art, which may be used to communicate signals among the above elements.

In some embodiments of the present disclosure, as shown in FIGs. 1A, 2, and 3, the tube carrier 10 is further provided with a first power source 2. The first power source 2 is configured to supply electrical power to electronic elements provided on the tube carrier 10 and needing to be supplied with power, such as the controller 40, the first wireless transceiver 80, and the regulator 30. The carrying portion 120 is provided with a second power source 3. The second power source 3 is configured to supply electrical power to electronic elements provided on the wearing member 100 and needing to be supplied with power, such as the auxiliary controller 70, the first wireless transceiver 80, the display 130, and the physical sign detector 60.

In the liquid transfer control device having the above structure according to some embodiments of the present disclosure, the tube carrier 10 is in wireless communication with the wearing member 100. During use, the wearing member 100 is tied to a human body and is configured to detect physical sign information of the human body; and a tube passes through the tube carrier 10 and is used for drawing blood, blood donation or liquid medicine transfusion related to the human body. In a case that the physical sign detector 60 in the wearing member 100 detects physical sign information (including a heart rate and/or a blood pressure) of the human body, the display 130 displays the physical sign information, and the controller 40 or the auxiliary controller 70 may determine whether the physical sign information is normal or not. In a case that the physical sign information is determined to be abnormal, the second alarm 52 gives an alarm prompt, and the controller 40 outputs a control signal to the regulator 30 to reduce or increase the cross-sectional area of the tube 1 arranged in the tube regulating mechanism 11, so as to allow the physical sign of the human body to gradually return to a normal range.

Further, the controller 40 may monitor the flow rate of the liquid in the tube 1 detected by the flow rate sensor 20. In a case that the controller 40 determines that the flow rate of the liquid in the tube 1 is abnormal, the controller induces the first alarm 51 to give an alarm, and output a control signal to the regulator 30. In response to the control signal, the regulator 30 regulates the flow rate of the liquid transferred in the tube 1 by regulating the cross-sectional area of the tube 1 arranged in the tube regulating mechanism 11, so as to control the flow rate of the liquid in the tube 1 within an normal value range and thus avoid a medical accident caused by the abnormality of the flow rate from happening.

Another aspect of some embodiments of the present disclosure further provides a liquid transfer apparatus including the liquid transfer control device having the above structure.

The liquid transfer apparatus further includes a tube made of a soft material and used for liquid transfer. In addition, the tube passes through the tube carrier, and at least a part of the tube passing through the tube carrier is arranged in the tube regulating mechanism.

Specifically, the liquid transfer apparatus may be an apparatus for drawing blood or blood donation, or may be an apparatus for transfusing liquid medicine to a human body.

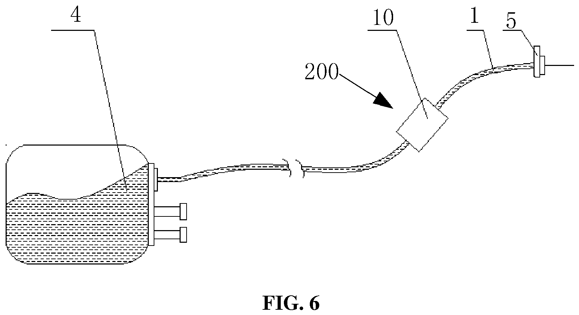

Taking as an example a case that the liquid transfer apparatus is an apparatus for drawing blood or blood donation, referring to FIG. 6 and FIGs. 1A and 2, the apparatus includes: a blood bag 4, a tube 1, a liquid transfer control device 200, and a blood collection needle 5. The tube 1 is connected to the blood bag 4 and the blood collection needle 5, and passes through the tube carrier 10 of the liquid transfer control device 200. An automatic regulation of a flow rate of blood in the tube 1 is realized through a flow rate sensor 20, a regulator 30, a controller 40 and the like arranged on the tube carrier 10.

Optionally, in conjunction with FIGs. 4 and 5, the liquid transfer apparatus according to some embodiments of the present disclosure further includes a wearing member 100. The wearing member is wirelessly connected to the tube carrier 10, and is configured to detect physical signs of a human body subjected to drawing blood or blood donation, and is configured to automatically regulate the flow rate of the blood in the tube 1 based on the physical sign information, and to give an alarm and display the flow rate.

Based on the drawings and the detailed description set forth above, those skilled in the art will be able to understand the liquid transfer apparatus employing the liquid transfer control device in some embodiments of the present disclosure, and further description of the liquid transfer apparatus will not be described in detail herein.

The liquid transfer apparatus according to some embodiments of the present disclosure may realize automatic intelligent control of blood donation, drawing blood, and liquid medicine transfusion, and avoid unnecessary medical accidents from happening during the blood donation, drawing blood or the liquid medicine transfusion in a medical process, and has great practical significance, and is simple in structure and easy to implement.

At least one of the above technical solutions of the embodiments of the present disclosure produces the following beneficial effects. In the liquid transfer control device according to some embodiments of the present disclosure, by arranging the tube to pass through the tube carrier, the regulator may regulate the cross-sectional area of the tube in the tube regulating mechanism and achieve ta purpose of regulating the flow rate in the tube; moreover, by monitoring the flow rate of the liquid in the tube by the flow rate sensor, the controller may control the regulator to regulate the cross-sectional area of the tube so as to regulate the flow rate of the liquid in the tube. The liquid transfer control device may be applied to a blood transfer tube used for drawing blood or blood donation, or may be applied to a liquid medicine transfer tube for transfusing liquid medicine to a human body; by monitoring a condition of the liquid transfer between the extracorporal environment and the intracorporal environment, the transfer rate of the liquid transfer may be controlled as appropriate so as to avoid a medical accident caused by the abnormality of the flow rate.

The above are optional embodiments of the present disclosure, and it should be noted that those skilled in the art may make various improvements and modifications without departing from the principle of the present disclosure, and such improvements and modifications shall be deemed to fall within the protection scope of the present disclosure.

## Claims

1. A liquid transfer control device, comprising:
a tube carrier, configured to accommodate at least one portion of a tube, wherein the tube is made of a soft material;
a flow rate sensor, configured to detect a flow rate of liquid transferred in the tube;
a regulator, configured to regulate the flow rate of the liquid transferred in the tube; and
a controller, coupled to the regulator and configured to output a control signal to the regulator based on the flow rate detected by the flow rate sensor,
wherein, the regulator is further configured to, in response to the control signal outputted by the controller, regulate the flow rate of the liquid in the tube accommodated in the tube carrier.

2. The liquid transfer control device according to claim 1, wherein the tube carrier further comprises a tube regulating mechanism, the tube regulating mechanism comprises a fluid container and an accommodation space, the accommodation space is arranged in the middle of the fluid container and configured to accommodate the at least one portion of the tube; and
the fluid container comprises a first surface facing towards the tube and made of an elastically deformable material.

3. The liquid transfer control device according to claim 2, wherein the regulator comprises a gas-pressure regulator, the gas-pressure regulator is arranged in the tube carrier and configured to regulate a cross-sectional area of the tube arranged in the accommodation space by regulating a gas pressure within the fluid container.

4. The liquid transfer control device according to claim 3, wherein the gas-pressure regulator comprises an air pump, a pressure regulating valve, and a gas transfer tube, the gas transfer tube has a first end communicated to an interior of the fluid container and a second end communicated to the air pump, wherein the pressure regulating valve is arranged on the gas transfer tube, and the air pump and the pressure regulating valve are configured to regulate the cross-sectional area of the tube by regulating the gas pressure within the fluid container.

5. The liquid transfer control device according to claim 2, wherein the fluid container is filled with liquid, the regulator comprises a hydraulic regulator, the hydraulic regulator is arranged in the tube carrier and configured to regulate the cross-sectional area of the tube arranged in the accommodation space by regulating a pressure of the liquid within the fluid container.

6. The liquid transfer control device according to claim 5, wherein the hydraulic regulator comprises a liquid storage tank, a hydraulic pump, a hydraulic value, a first liquid transfer tube, and a second liquid transfer tube; the hydraulic pump is connected to the liquid storage tank; the first liquid transfer tube has a first end communicated to the hydraulic pump and a second end communicated to an interior of the fluid container; the second liquid transfer tube has a first end communicated to the liquid storage tank and a second end communicated to the interior of the fluid container; the hydraulic valve is arranged on the second liquid transfer tube; and the hydraulic pump and the hydraulic valve are configured to regulate the cross-sectional area of the tube by regulating the pressure of the liquid within the fluid container.

7. The liquid transfer control device according to any one of claims 1 to 6, wherein the controller is further configured to output the control signal to the regulator in response to a detection result that the flow rate detected by the flow rate sensor falls outside a first predefined value range.

8. The liquid transfer control device according to any one of claims 1 to 7, further comprising:
a first alarm, arranged on the tube carrier and configured to send first alarm prompt information;
wherein the controller is further configured to, in response to a detection result that the flow rate detected by the flow rate sensor falls outside the first predefined value range, send a first alarm signal to the first alarm, the first alarm is further configured to send the first alarm prompt information in response to the first alarm signal.

9. The liquid transfer control device according to any one of claims 1 to 8, further comprising:
a physical sign detector configured to detect physical sign information of a human body connected to the tube;
wherein the controller is further configured to acquire the physical sign information detected by the physical sign detector, and output the control signal to the regulator based on the physical sign information, so that the regulator regulates the cross-sectional area of the tube accommodated in the tube carrier in response to the control signal.

10. The liquid transfer control device according to claim 9, wherein the controller is further configured to output the control signal to the regulator in response to a detection result that the physical sign information detected by the physical sign detector falls outside a second predefined value range.

11. The liquid transfer control device according to claim 9 or 10, further comprising:
a second alarm configured to send second alarm prompt information;
wherein the controller is further configured to, in response to the detection result that the physical sign information detected by the physical sign detector falls outside the second predefined value range, send a second alarm signal to the second alarm; the second alarm is further configured to send the second alarm prompt information in response to the second alarm signal.

12. The liquid transfer control device according to any one of claims 9 to 11, wherein the physical sign detector is arranged on a wearing member, the wearing member is fixable onto a human body, and in a case that the wearing member is fixed to the human body, the physical sign detector is capable of detecting the physical sign information of the human body, and
the liquid transfer control device further comprises:
a first wireless transceiver coupled to the physical sign detector and arranged on the wearing member; and
a second wireless transceiver coupled to the controller and arranged on the tube carrier;
wherein the physical sign information detected by the physical sign detector is transmitted to the second wireless transceiver via the first wireless transceiver and the controller acquires the physical sign information from the second wireless transceiver.

13. The liquid transfer control device according to any one of claims 9 to 12, further comprising:
a display configured to acquire and display the flow rate detected by the flow rate sensor and the physical sign information detected by the physical sign detector.

14. The liquid transfer control device according to claim 13, wherein the display is a touch display screen and configured to enable a user to input at least one of the first predefined value range of the flow rate or the second predefined value range of the physical sign information.

15. The liquid transfer control device according to claim 1, wherein the tube is a blood transfer tube used for drawing blood or blood donation, or
the tube is a liquid-medicine transfer tube used to transfuse liquid medicine to a human body.

16. The liquid transfer control device according to any one of claims 2 to 6, wherein at least a portion of the flow rate sensor is inserted into the fluid container; or
the flow rate sensor is arranged outside the fluid container.

17. The liquid transfer control device according to any one of claims 9 to 13, wherein the physical sign sensor comprises at least one of a body temperature detector, a pulse detector, or a blood pressure detector.

18. The liquid transfer control device according to claim 12, wherein the wearing member further comprises an auxiliary controller, the auxiliary controller is configured to be coupled to the first wireless transceiver and to communicate with the controller via the first wireless transceiver.

19. The liquid transfer control device according to claim 1, wherein the regulator comprises a cross-section regulator, the cross-section regulator is configured to regulate the flow rate of the liquid in the tube in response to the control signal from the controller.

20. The liquid transfer control device according to claim 19, wherein the cross-section regulator comprises a pneumatic actuator, an air pump, and a gas transfer tube, the gas transfer tube connects the pneumatic actuator with the air pump, and the air pump is configured to control the pneumatic actuator to regulate the cross-sectional area of the tube in response to the control signal from the controller; or
the cross-section regulator comprises a hydraulic regulator, a hydraulic pump, and a liquid transfer tube, the liquid transfer tube connects the hydraulic regulator with the hydraulic pump, and the hydraulic pump is configured to control the hydraulic regulator to regulate the cross-sectional area of the tube in response to the control signal from the controller; or
the cross-section regulator comprises an electric actuator and a motor, the motor is configured to control the electric actuator to regulate the cross-sectional area of the tube in response to the control signal from the controller.

21. A liquid transfer apparatus, comprising:
the liquid transfer control device according to any one of claims 1 to 20; and
a tube made of a soft material and configured for transferring liquid, wherein at least one portion of the tube is accommodated in the tube carrier.
